# FASCICULE DE BREVET EUROPEEN

(11) **EP 2 763 798 B1**
(45) Date de publication et mention de la délivrance du brevet: **03.01.2018**
(21) Numéro de dépôt: 12778662.2
(22) Date de dépôt: 05.10.2012
(51) Int. Cl.: B05D 1/18, B05D 3/14, C07D 257/00, C07D 339/00, G11B 9/00, G11C 11/00, C07D 409/00

(54) **PROCÉDÉ DE FONCTIONNALISATION D'UN SUBSTRAT SOLIDE AUTRE QU'UN SUBSTRAT EN OR PAR DES COMPOSÉS CHIMIQUES SPÉCIFIQUES**
VERFAHREN ZUR FUNKTIONALISIERUNG EINES FESTEN SUBSTRATS OHNE GOLD DURCH SPEZIFISCHE CHEMISCHE VERBINDUNGEN
METHOD FOR FUNCTIONALIZING A SOLID SUBSTRATE, OTHER THAN A SUBSTRATE MADE OF GOLD, VIA SPECIFIC CHEMICAL COMPOUNDS

(30) Priorité: 06.10.2011 FR 1159045
(43) Date de publication de la demande: 13.08.2014
(73) Titulaire: Commissariat à l'Énergie Atomique et aux Énergies Alternatives, 75015 Paris (FR)
(72) Inventeur: JALAGUIER, Eric, F-38410 Saint Martin D'uriage (FR); BUCKLEY, Julien, F-38000 Grenoble (FR); CHEVALIER, Xavier, F-38100 Grenoble (FR); ROYAL, Guy, F-73800 Cruet (FR)
(74) Mandataire: Ahner, Philippe
(86) Numéro de dépôt international: PCT/EP2012/069689
(87) Numéro de publication internationale: WO 2013/050512

(56) Documents cités:
- EP-A1- 1 215 205
- EP-A1- 1 703 572
- WO-A1-02/071151
- ULMAN A: "Formation and Structure of Self-Assembled Monolayers", 1 janvier 1996 (1996-01-01), CHEMICAL REVIEWS, AMERICAN CHEMICAL SOCIETY, US, PAGE(S) 1533 - 1554, XP002239617, ISSN: 0009-2665 page 1539, colonne 2, ligne 1 - ligne 5; figure 8
- LON A. PORTER ET AL: "Gold and Silver Nanoparticles Functionalized by the Adsorption of Dialkyl Disulfides", LANGMUIR, vol. 14, no. 26, 1 décembre 1998 (1998-12-01), pages 7378-7386, XP055030888, ISSN: 0743-7463, DOI: 10.1021/la980870i
- LARSSON M L ET AL: "Direct observation of a self-assembled monolayer of heptyl xanthate at the germanium/water interface: a polarized FTIR study", JOURNAL OF COLLOID AND INTERFACE SCIENCE, ACADEMIC PRESS, NEW YORK, NY, US, vol. 273, no. 2, 15 mai 2004 (2004-05-15), pages 345-349, XP027132605, ISSN: 0021-9797 [extrait le 2004-04-10]

## Description

### DOMAINE TECHNIQUE

La présente invention a trait à un procédé de préparation d'un substrat autre qu'un substrat en or fonctionnalisé par une couche constituée de composés chimiques, ces substrats ainsi préparés pouvant entrer dans la constitution de dispositifs de mémoire et en particulier de dispositifs de mémoire à base de silicium, notamment lorsque les composés chimiques comportent au moins un groupe de stockage de charges.

Un des domaines d'application de l'invention est ainsi celui des dispositifs de mémoire utilisant des composés chimiques comme composés de stockage de charges (ces composés pouvant être également qualifiés de mémoires moléculaires).

### ÉTAT DE LA TECHNIQUE ANTÉRIEURE

Les dispositifs de mémoire utilisant des mémoires moléculaires reposent sur le principe du stockage de charges au niveau d'un ou plusieurs composés chimiques formant des complexes de coordination avec un ou plusieurs éléments métalliques (ces complexes constituant, en tant que tels, des mémoires moléculaires). Afin de pouvoir stocker une ou plusieurs charges, ces complexes métalliques doivent avoir des propriétés redox bien définies et exister sous au moins deux états d'oxydation distincts, lesquels états d'oxydation correspondent à deux états de charge, un de ces états étant l'état « effacé » et l'autre état étant l'état « écrit ». Le passage d'un état à l'autre se fait par transfert de charge *via* un mécanisme de réaction d'oxydoréduction réversible, sous l'effet d'un stimulus extérieur pouvant être de nature chimique (pH, réactif), photochimique (tel qu'une irradiation à une longueur d'onde donnée) ou électrochimique (tel que l'application d'un potentiel d'oxydoréduction donnée, ce potentiel s'échelonnant classiquement de +2 à -2V). Ces dispositifs de mémoire à stockage de charges présentent des avantages, tels que la réduction de taille du dispositif et l'utilisation de tensions d'alimentation faibles. Qui plus est, l'utilisation de complexes présentant plus de deux états redox, peut permettre d'élaborer des dispositifs de mémoire multi-bits.

Toutefois, pour assurer toutes ces fonctionnalités, les complexes métalliques susmentionnés doivent être fixés, de façon stable, sur un substrat solide, classiquement conducteur de l'électricité, ce qui implique souvent de fonctionnaliser de façon appropriée lesdits complexes et/ou d'appliquer un traitement de surface spécifique au substrat, afin de pouvoir fixer lesdits complexes au substrat au moyen de liaisons chimiques, la fixation de ces complexes sur un substrat ne devant pas imputer les propriétés de commutation de ceux-ci après fixation.

Ainsi, à titre d'exemple, il a été procédé à la fonctionnalisation de substrats métalliques par des composés cyclames en utilisant des groupes de fixation du type pyrrole, avec toutefois, selon ce mode de réalisation, l'inconvénient d'engendrer une fonctionnalisation sous forme de multicouches plus ou moins organisées, ce qui nuit à l'uniformité des propriétés du substrat ainsi fonctionnalisé.

Il a été procédé également à la fonctionnalisation de substrats métalliques en or par des composés cyclames complexés avec du cuivre métallique porteurs de groupes de fixation du type thiol, cette fonctionnalisation consistant uniquement à tremper le substrat en or dans une solution comprenant lesdits composés, lesdits composés s'organisant spontanément sous forme de couches auto-assemblées sur le substrat en or, avec pour inconvénient majeur la nature même du substrat. En effet, l'or constitue un contaminant métallique pour les dispositifs de mémoire impliquant du silicium, ce qui rend son utilisation rédhibitoire dans ce contexte.

Les inventeurs de la présente invention, eu égard aux problématiques susmentionnées, se sont fixé comme objectif de proposer un nouveau procédé de fonctionnalisation de substrats conducteurs de l'électricité par des composés chimiques, ces substrats pouvant être destinés à être utilisés dans des dispositifs de mémoire, n'impliquant pas l'utilisation de l'or pour la constitution du substrat et présentant les avantages suivants :
- l'organisation des composés chimiques sous forme de couches à la surface du substrat, ce qui induit une uniformité de propriétés du substrat ainsi fonctionnalisé ; et
- lorsque lesdits composés chimiques sont des complexes métalliques, la conservation des propriétés de commutation desdits complexes après fixation sur le substrat.

### EXPOSÉ DE L'INVENTION

Les inventeurs ont ainsi découvert, de manière surprenante, qu'en utilisant des composés comprenant un groupe spécifique apte après électrooxydation à se fixer spontanément à la surface d'un substrat conducteur de l'électricité, qui ne comprend pas d'or, il est possible de surmonter les inconvénients susmentionnés et en particulier de fonctionnaliser un tel substrat ne comportant pas d'or par une couche de ces composés chimiques.

L'invention a ainsi trait à un procédé de fonctionnalisation d'un substrat conducteur de l'électricité qui n'est pas un substrat en or par une couche de composés chimiques, comprenant les étapes suivantes :
- une étape de mise en contact du substrat conducteur de l'électricité avec des composés chimiques spécifiques tels que définis ci-dessous comprenant au moins un groupe terminal disulfure;
- une étape d'électrooxydation du groupe terminal disulfure desdits composés chimiques moyennant quoi lesdits composés chimiques forment une couche à la surface du substrat conducteur de l'électricité.

Par fonctionnalisation, on entend un procédé d'élaboration d'une couche de molécules de composés chimiques susmentionnés à la surface d'un substrat conducteur de l'électricité.

En particulier, lesdits composés chimiques, outre la présence d'au moins un groupe terminal disulfure comprennent au moins un groupe de stockage de charges, ledit groupe de stockage de charges et ledit groupe terminal disulfure étant éventuellement séparés par au moins un groupe espaceur organique, ces composés répondant à la formule générale (I) suivante : 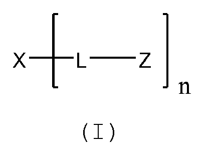 dans laquelle :
- X représente un groupe de stockage de charges ;
- L représente une liaison simple ou un groupe espaceur organique ;
- Z représente un groupe disulfure ; et
- n est un entier allant de 1 à 6.

Le groupe de stockage de charges peut être classiquement un groupe organique complexant au moins un élément métallique présentant au moins deux degrés d'oxydation, moyennant quoi il constitue un complexe de coordination avec le ou lesdits éléments métalliques.

Un tel complexe de coordination se matérialise ainsi par un composé chimique comprenant des fonctions établissant des liaisons dites « liaisons de coordination » avec un élément métallique, ces fonctions comprenant classiquement un doublet libre, qui va venir occuper une orbitale vide de l'élément métallique en question.

De tels groupes de stockage de charges sont des groupes polyazacycloalcanes, soit, en d'autres termes, des groupes cycloalcanes comprenant dans leur cycle plusieurs atomes d'azote, un exemple spécifique de tels groupes étant un groupe tétraazacycloalcane, ces groupes étant complexés à au moins un élément métallique.

Des groupes tétraazacycloalcanes conformes à l'invention répondent à l'une des formules (II) à (IV) suivantes : 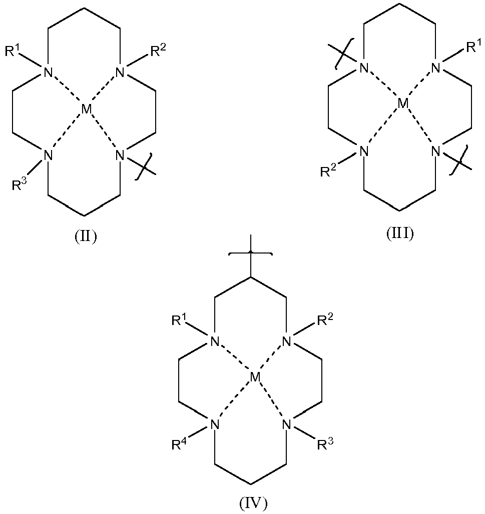 dans lesquelles :
- R¹, R², R³ et R⁴ représentent, indépendamment l'un de l'autre, un groupe alkyle, aryle, alkylaryle, un atome d'halogène, un groupe (alkyl)métallocène, lesdits groupes pouvant être perfluorés;
- M est un élément métallique présentant au moins deux degrés d'oxydation,
les accolades indiquant l'endroit par lequel ces groupes sont liés au groupe disulfure susmentionné *via* éventuellement un groupe espaceur organique.

Par groupe alkyle, on entend classiquement, dans ce qui précède et ce qui suit, un groupe alkyle linéaire ou ramifié pouvant comprendre de 1 à 20 atomes de carbone ou cyclique pouvant comprendre de 3 à 20 atomes de carbone. On peut citer, à titre d'exemples, le groupe méthyle, éthyle, n-propyle, i-propyle, n-butyle, n-dodécanyle, i-butyle, t-butyle, cyclopropyle, cyclohexyle.

Par groupe aryle, on entend, généralement, dans ce qui précède et ce qui suit, un groupe aryle pouvant comprendre de 6 à 20 atomes de carbone. On peut citer, à titre d'exemples, le groupe benzyle, naphtyle, biphényle.

Par groupe alkylaryle, on entend, généralement, dans ce qui précède et ce qui suit, un groupe aryle de même définition que celle donnée ci-dessus, ledit groupe étant substitué par au moins un groupe alkyle de définition identique à celle donnée ci-dessus.

Par groupe (alkyl)métallocène, on entend un groupe comprenant au moins un atome métallique pris en sandwich entre au moins deux structures organiques cycliques conjuguées, ce groupe métallocène pouvant être lié au cyclame via un groupe alkylène, auquel cas on parlera de groupe alkylferrocène.

Par perfluoré, on entend un groupe dont tous les atomes d'hydrogène sont substitués par des atomes de fluor.

En particulier, lorsque les groupes tétraazacycloalcanes appartiennent à la catégorie des groupes de formule (II) susmentionnée :
- R¹, R², R³ peuvent représenter un groupe alkyle tel que défini ci-dessus, un tétraazacycloalcane répondant à cette spécificité étant celui répondant à la formule (V) suivante : 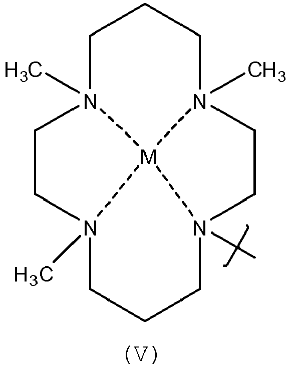 dans laquelle M correspond à la même définition que celle donnée ci-dessus ; ou
- R¹ peut représenter un groupe alkyle et R² et R³ peuvent représenter un groupe (alkyl)métallocène (symbolisé ci-après -CH₂-T), un groupe tétraazacycloalcane répondant à cette spécificité étant celui répondant à la formule (VI) suivante : 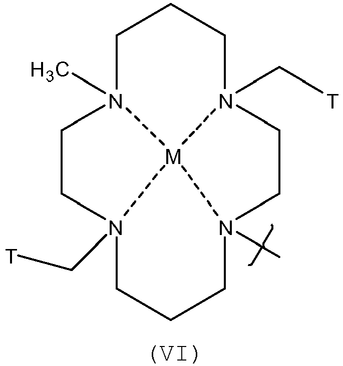 T représentant un groupe ferrocène, à savoir deux groupes cyclopentadiènes prenant en sandwich un atome de fer.

Le ou les éléments métalliques M mentionnés ci-dessus peuvent être des éléments métalliques de transition, tels que Cr, Mn, Fe, Co, Ni, Cu.

Plus particulièrement, l'élément métallique peut être le cuivre, notamment le cuivre, par exemple, au degré d'oxydation +I ou +II.

Les groupes tétraazacycloalcanes, tels que ceux définis ci-dessus, sont particulièrement avantageux en tant que groupe de stockage de charges, notamment lorsqu'ils complexent du cuivre et ce pour les raisons suivantes :
- ils présentent deux états redox discrets (+I et +II), chacun de ces deux états présentant une très grande stabilité ;
- ils présentent un comportement électrochimique montrant la présence d'un phénomène d'hystérèse.

Concernant le premier point susmentionné, sans être lié par la théorie, la stabilité résulte du fait que la modification de la charge du complexe métallique par oxydation ou réduction de l'élément métallique complexé induit un mouvement à l'échelle moléculaire, c'est-à-dire un changement de géométrie du complexe. Ce mouvement est réversible et est à l'origine de la très grande stabilisation à la fois chimique et électrochimique de la charge injectée initialement. Ces groupes sont ainsi des molécules redox bistables existant sous deux états redox très stables (un état redox étant associé à l'état « 0 » et l'autre état redox étant associé à l'état « 1 »), chacun de ces états présentant un temps de rétention de charge très élevé, le passage d'une forme à l'autre s'effectuant de manière réversible et reproductible par simple transfert d'électrons. Il est noté, également, que les états « 0 » et « 1 » de ces groupes sont particulièrement stables en présence d'air, ce qui implique qu'ils peuvent être stockés sans précautions particulières.

Concernant le deuxième point susmentionné, sans être lié par la théorie, les deux états « 0 » et « 1 » de ces groupes présentent des potentiels d'oxydoréduction bien distincts (pouvant être dénommés respectivement E⁰_{Etat0} et E⁰_{Etat1}) et, à un potentiel donné compris entre E⁰_{Etat0} et E⁰_{Etat1}, lesdits groupes restent sous leur état « 0 » ou leur état « 1 », selon qu'ils ont été préalablement réduits ou oxydés, le passage d'un état à l'autre nécessitant l'application d'une surtension.

Comme mentionné plus haut, le groupe de stockage de charges (par exemple, X, dans la formule (I)) et le groupe disulfure (par exemple, Z, dans la formule (I)) peuvent être séparés par un groupe espaceur organique.

Ledit groupe espaceur organique peut être un groupe hydrocarboné, se présentant sous forme d'une chaîne linéaire ou ramifiée, dans laquelle peuvent venir s'intercaler un ou plusieurs groupes de liaison.

Il peut s'agir, plus particulièrement, d'un groupe alkylène, pouvant comporter de 1 à 12 atomes de carbone, dans lequel peuvent venir s'intercaler un ou plusieurs groupes de liaison.

Ces groupes de liaison peuvent être des groupes -(C=O)-, -(C=O)O-, -SO₂, des groupes amides.

Un exemple spécifique de groupe alkylène peut être un groupe de formule (VII) suivante :

-(CH₂)₄-NH-CO-(CH₂)₄- (VII)

Le groupe disulfure conforme à l'invention (qui peut être sous forme racémique ou sous une forme énantiomère pure) peut être, de préférence, un groupe disulfure cyclique (ce qui signifie, en d'autres termes, que le groupe -S-S- est incorporé dans un cycle), pouvant répondre à la formule (VIII) suivante : 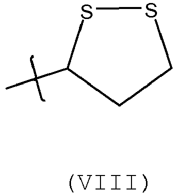 l'accolade indiquant l'endroit par lequel le groupe disulfure est lié au groupe de stockage de charges via éventuellement un groupe espaceur organique.

Des composés organiques conformes à l'invention peuvent correspondre aux composés de formules (IX) et (X) suivantes : 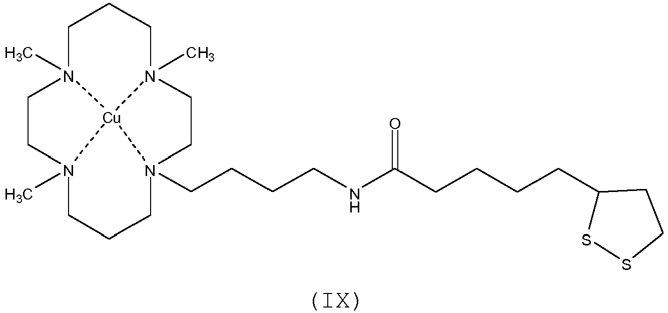 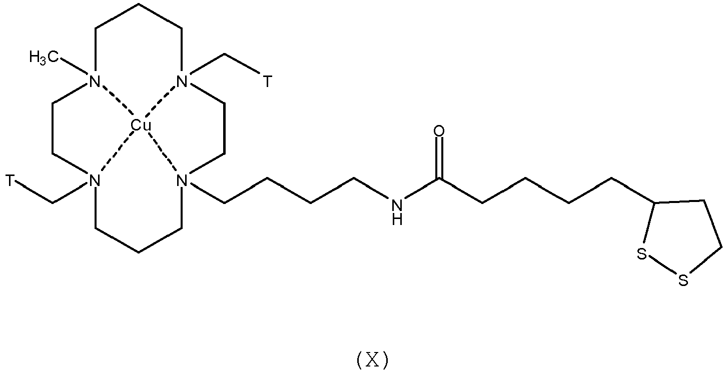 avec T étant tel que défini ci-dessus.

Comme mentionné plus haut, le substrat destiné à être fonctionnalisé est un substrat conducteur de l'électricité qui n'est pas un substrat en or, ce substrat pouvant être un substrat en un matériau choisi parmi le carbone, un métal noble, tel que le platine, un oxyde métallique, tel qu'un oxyde d'indium-étain, un oxyde de nickel, un métal de transition, tel que le nickel, et, en particulier le carbone vitreux.

Conformément à l'invention, le substrat est porté à un potentiel permettant l'électrooxydation du groupe disulfure, qui engendre spontanément la fixation d'une couche de composés susmentionnés à la surface du substrat.

Selon un premier mode de réalisation, cette étape peut consister à soumettre le milieu comprenant le substrat et le composé susmentionné à un cyclage mis en oeuvre dans une gamme de potentiels nécessaire à l'électrooxydation du groupe disulfure.

En d'autres termes, cette gamme de potentiel est une gamme de potentiels comprenant le potentiel d'oxydation du groupe disulfure (qui peut être de l'ordre de +0,8 V par rapport à Ag⁰/Ag⁺ (10 mmol.L⁻¹ dans l'acétonitrile), la répétition du nombre de cycles se traduisant par une diminution de l'intensité anodique au potentiel d'oxydation susmentionné, ce qui indique que les groupes disulfures sont consommés de manière irréversible.

Selon un deuxième mode de réalisation, cette étape peut consister à soumettre le milieu comprenant le substrat et le composé susmentionné à un potentiel contrôlé apte à permettre l'oxydation du groupe disulfure (ce potentiel pouvant être de l'ordre de +0,8 V comme mentionné ci-dessus).

Ce potentiel contrôlé peut être appliqué pendant un temps nécessaire pour obtenir une diminution, en tout ou partie, de l'intensité anodique au potentiel d'oxydation susmentionné, ce qui indique que les groupes disulfures sont consommés, en tout ou partie, de manière irréversible, cette diminution pouvant être déterminée par voltampérométrie cyclique par l'homme du métier.

Les auteurs de la présente invention ont pu constater, de manière surprenante, qu'en oxydant par voie électrochimique le groupe disulfure des composés susmentionnés, ces derniers se fixent spontanément et de façon concomitante à la surface de substrats ne comportant pas d'or, sous forme d'au moins une couche, ce qui en fait des substrats fonctionnalisés par une couche de composés chimiques (organiques ou organométalliques), tels que définis ci-dessus, ces substrats ainsi fonctionnalisés pouvant ensuite être utilisés comme substrats à interrupteurs moléculaires lorsque les composés ainsi fixés comprennent un groupe apte à assurer cette fonction d'interrupteur moléculaire.

Les caractéristiques de substrats et de composés organiques sont identiques à celles déjà mentionnées dans le cadre de la description du procédé ci-dessus.

Ainsi, l'invention a trait également à un substrat susceptible d'être obtenu par le procédé tel que défini ci-dessus.

De tels substrats, notamment lorsqu'ils comportent une couche à base de composés chimiques comportant au moins un groupe de stockage de charges, peuvent entrer avantageusement dans la constitution de cellules de mémoire destinées à entrer dans la constitution de dispositifs électroniques, par exemple, des dispositifs de mémoire capacitive.

On entend par "mémoire capacitive" toute structure apte à stocker des charges, intégrant ou non une capacité en tant que telle. L'invention s'applique particulièrement bien aux mémoires capacitives de type flash, qui stockent des charges dans un transistor, ou de type DRAM qui stockent des charges dans une capacité.

Ainsi, l'invention peut avoir trait à une cellule capacitive de mémoire comprenant au moins un substrat fonctionnalisé conforme à l'invention, qui pourrait, par exemple, être inclus dans une architecture de matrice mémoire dite « DRAM », pour Dynamic Random Access Memory ou Mémoire dynamique à accès direct en français.

Une cellule mémoire DRAM classique est constituée d'un transistor MOS (Oxyde métallique semi-conducteur) d'accès et d'une capacité de stockage, la capacité pouvant être constituée de deux électrodes séparées par un électrolyte, et des molécules à stockage de charges encapsulées dans l'électrolyte et couplées électroniquement à une des deux électrodes.

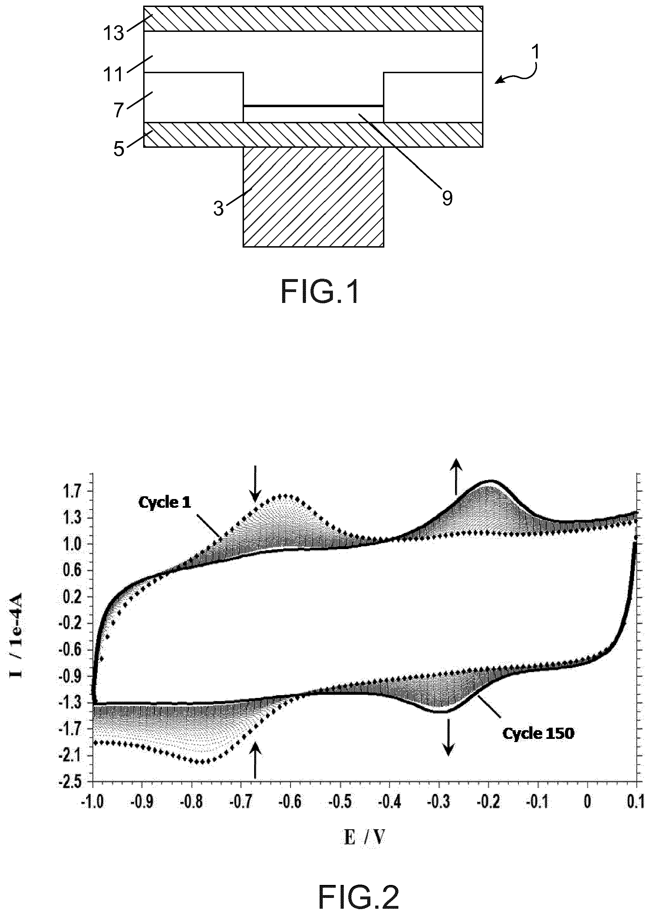

Une telle capacité peut être en configuration dite « configuration stack » comme illustré sur la figure 1 jointe en annexe (référence 1 sur la figure), qui comprend :
- un plot conducteur 3, qui peut être en un métal ou alliage métallique (par exemple, en W, Al, Cu), en siliciure métallique ou tous autres matériaux utilisés dans les interconnexions électriques du procédé Back-End de la technologie CMOS des circuits intégrés, ce plot pouvant présenter une épaisseur de quelques centaines de nanomètres (nm) à 1 µm ;
- une électrode de travail 5, par exemple une électrode métallique (telle qu'une électrode en W, Al, Ti ou Cu), une électrode en nitrure (telle qu'une électrode en TiN, TaN) ou une électrode en matériau semi-conducteur (telle qu'une électrode en Si, Ge, SiGe), cette électrode pouvant présenter une épaisseur de quelques dizaines de nm à quelques centaines de nm ;
- une couche isolante 7, telle qu'une couche en oxyde de silicium (SiO₂) ou en nitrure de silicium (Si₃N₄) déposée pour passiver l'électrode de travail 5, cette couche isolante pouvant présenter une épaisseur allant de quelques dizaines de nm à quelques centaines de nm ;
- une couche de composés chimique 9 conforme à l'invention, cette couche étant en contact avec l'électrode de travail *via* sa zone active, l'ensemble formant un substrat fonctionnalisé conforme à l'invention ;
- une couche électrolytique 11, de préférence une couche électrolytique solide, par exemple, un gel électrolytique (comme un gel en polyméthacrylate de méthyle dopé au perchlorate de lithium ;
- une électrode métallique 13 jouant le rôle d'électrode de référence ou de contre-électrode, par une électrode en Cu, Ag et/ou Pt, cette électrode pouvant présenter une épaisseur allant de quelques dizaines de nm à quelques centaines de nm.

La couche électrolytique susmentionnée, lorsqu'elle est constituée d'un gel électrolytique peut être préparée par dissolution d'un électrolyte choisi parmi l'hexafluorophosphate de tétrabutylammonium (TBAPF₆), LiPF₆, LiClO₄ et LiBF₄ dans un solvant choisi parmi le carbonate de propylène, le sulfolane, le 3-méthyle-2-oxazolidinone, le 4-méthyle-2-pentanone. La viscosité du gel peut être contrôlée par ajout d'un matériau polymère de haut poids moléculaire choisi parmi le poly(fluorure de vinylidène)-hexafluoropropylène, le méthacrylate de 2-hydroxyéthyle, l'acrylonitrile, le méthacrylate de méthyle, l'oxyde de polyéthylène, les polyphosphazènes.

Alternativement, la couche électrolytique peut être remplacée par un liquide ionique dont la viscosité est contrôlée par un matériau polymère tel que décrit ci-dessus.

Les liquides ioniques qui sont généralement utilisés dans le cadre de l'invention sont le tétrafluoroborate de 1-éthyle-3-méthyle imidazolium, le trifluorométhanesulfonate du 1-éthyle-3-méthyle imidazolium, l'hexafluorophosphate de 1-(1-butyl)-3-méthylimidazolium ou le tétrafluoroborate de 1-butylpyridinium.

Le dépôt du gel électrolytique pourra être réalisé par les méthodes usuelles utilisées dans les procédés sol-gel : trempage-retrait (« dipping »), tournette (« spin-coating »), enduction laminaire, nébulisation (spraying).

Parmi les composés organiques susceptibles d'être utilisés pour la mise en oeuvre du procédé susmentionné, certains sont nouveaux et correspondent à des composés organiques spécifiques tels que définis ci-dessous comprenant au moins un groupe de stockage de charges et au moins un groupe terminal disulfure, ledit groupe de stockage de charges et ledit groupe disulfure étant éventuellement séparé par un groupe espaceur organique.

Ces composés répondent à la formule générale (I) suivante :

X-[-L-Z]ₙ (I)

dans laquelle :
- X représente un groupe de stockage de charges tel que défini ci-dessous ;
- L représente une liaison simple ou un groupe espaceur organique ;
- Z représente un groupe disulfure ; et
- n est un entier allant de 1 à 6.

Le groupe de stockage de charges peut être classiquement un groupe organique complexant au moins un élément métallique présentant au moins deux degrés d'oxydation, moyennant quoi il constitue un complexe de coordination avec le ou lesdits éléments métalliques.

De tels groupes de stockage de charges sont des groupes polyazacycloalcanes, soit, en d'autres termes, des groupes cycloalcanes comprenant dans leur cycle plusieurs atomes d'azote, un exemple spécifique de tels groupes étant un groupe tétraazacycloalcane, ces groupes étant complexés à au moins un élément métallique.

Des groupes tétraazacycloalcanes conformes à l'invention répondent à l'une des formules (II) à (IV) suivantes : 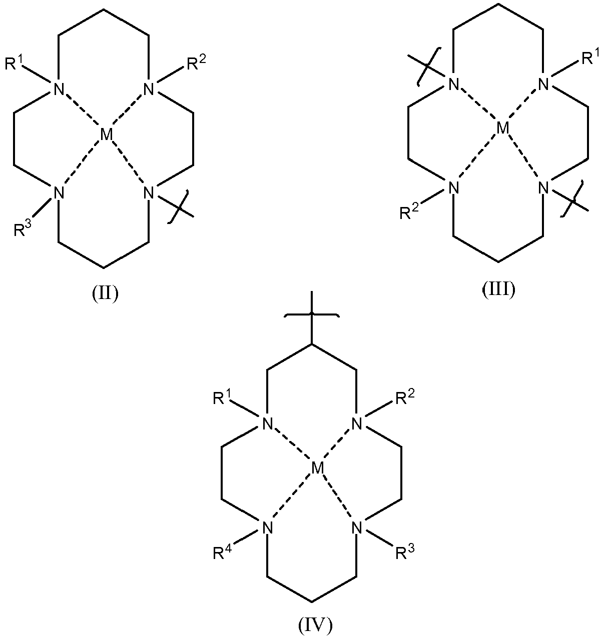 dans lesquelles :
- R¹, R², R³ et R⁴ représentent indépendamment un groupe alkyle, aryle, alkylaryle, un atome d'halogène, lesdits groupes pouvant être perfluorés;
- M est un élément métallique présentant au moins deux degrés d'oxydation,
les accolades indiquant l'endroit par lequel ces groupes sont liés au groupe disulfure *via* éventuellement un groupe espaceur organique.

En particulier, lorsque les groupes tétraazacycloalcanes appartiennent à la catégorie des groupes de formule (II) susmentionnée :
- R¹, R², R³ peuvent représenter un groupe alkyle tel que défini ci-dessus, un tétraazacycloalcane répondant à cette spécificité étant celui répondant à la formule (V) suivante : 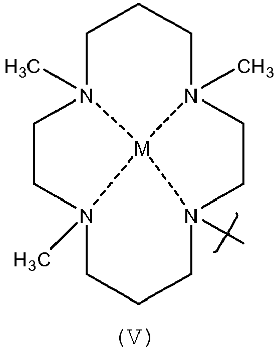 dans laquelle M correspond à la même définition que celle donnée ci-dessus ; ou
- R¹ peut représenter un groupe alkyle et R² et R³ peuvent représenter un groupe (alkyl)métallocène, un groupe tétraazacycloalcane répondant à cette spécificité étant celui répondant à la formule (VI) suivante : 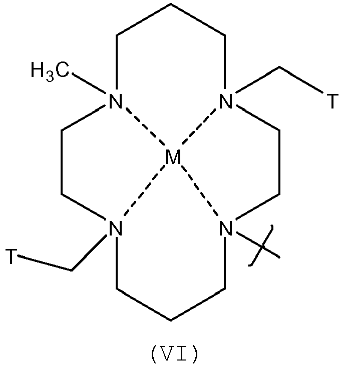
T représentant un groupe ferrocène, à savoir deux groupes cyclopentadiènes prenant en sandwich un atome de fer.

Le ou les éléments métalliques mentionnés ci-dessus peuvent être des éléments métalliques de transition, tels que Cr, Mn, Fe, Co, Ni, Cu.

Plus particulièrement, l'élément métallique peut être le cuivre, et plus particulièrement le cuivre au degré d'oxydation (II).

Les avantages de ce type de composés mentionnés ci-dessus dans la partie relative à la description du procédé peuvent être repris ici.

Comme mentionné plus haut, le groupe de stockage de charges (par exemple, X, dans la formule (I)) et le groupe disulfure (par exemple, Z, dans la formule (I)) peuvent être séparés par un groupe espaceur organique.

Ledit groupe espaceur organique peut être un groupe hydrocarboné, se présentant sous forme d'une chaîne linéaire ou ramifiée, dans lequel peuvent venir s'intercaler un ou plusieurs groupes de liaison.

Il peut s'agir, plus particulièrement, d'un groupe alkylène, pouvant comporter de 1 à 12 atomes de carbone, dans lequel peuvent venir s'intercaler un ou plusieurs groupes de liaison.

Ces groupes de liaison peuvent être des groupes -(C=O)-, -(C=O)O-, -SO₂, des groupes amides.

Un exemple spécifique de groupe alkylène peut être un groupe de formule (VII) suivante :

-(CH₂)₄-NH-CO-(CH₂)₄- (VII)

Le groupe disulfure conforme à l'invention peut être un groupe disulfure cyclique (ce qui signifie, en d'autres termes, que le groupe -S-S- est incorporé dans un cycle), pouvant répondre à la formule (VII) suivante : 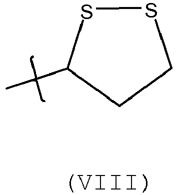 l'accolade indiquant l'endroit par lequel le groupe disulfure est lié au groupe de stockage de charges via éventuellement un groupe espaceur.

Des composés organiques conformes à l'invention peuvent être choisis parmi les composés de formules (IX) et (X) suivantes : 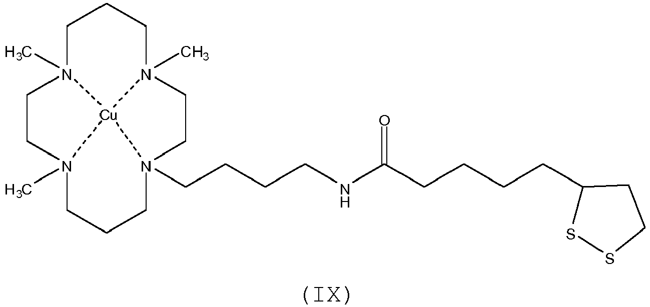 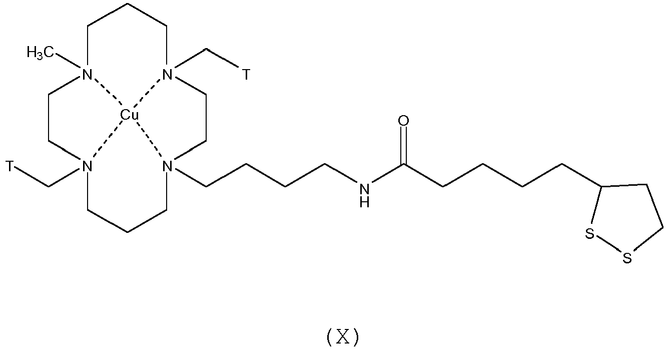 avec T étant tel que défini ci-dessus.

L'invention va être à présent décrite en référence au mode de réalisation particulier ci-dessous donné à titre illustratif et non limitatif.

### BRÈVE DESCRIPTION DES DESSINS

La figure 1 représente une configuration spécifique d'un dispositif capacitif à stockage de charges conforme à l'invention.
La figure 2 représente un voltampérogramme obtenu dans le cadre de l'exemple 2.

### EXPOSÉ DÉTAILLÉ DE MODES DE RÉALISATION PARTICULIERS

Dans cette partie, vont être illustrées :
- la préparation d'un composé organique spécifique comprenant un groupe terminal disulfure (Exemple 1) ;
- la fonctionnalisation d'une électrode en carbone vitreux par ce composé et l'étude du système obtenu par voltammogramme (Exemple 2).

### EXEMPLE 1

Cet exemple illustre la préparation d'un composé de formule (XI) suivante : 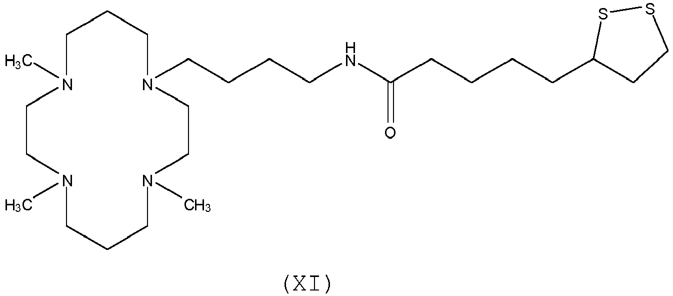 qui se déroule en trois étapes :
- la synthèse du 1,4,8,11-tétraazatricyclo[9,3,1,1]hexadécane de formule (XII) suivante : 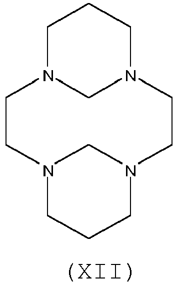
- à partir du composé de formule (XII), la synthèse du sel iodure de méthyl-4,11-diazoniatricyclo[9,3,1,1]hexadecane de formule (XIII) suivante : 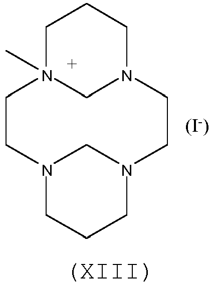
- à partir du composé de formule (XIII), la synthèse du (Propan-3-nitrile)-4,8,11-triméthyl-1,4,8,11-tétraazacyclotétradécane de formule (XIV) suivante : 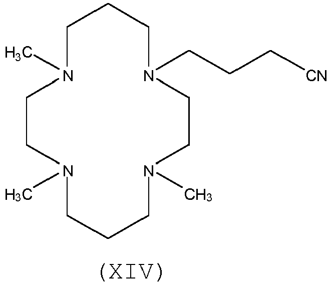
- à partir du composé de formule (XIV), la synthèse du (Butan-4-amine)-4,8,11-triméthyl-1,4,8,11-tétraazacyclotétradécane de formule (XV) suivante : 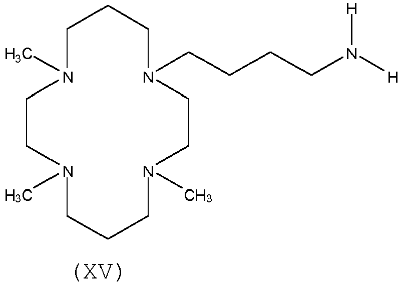
- à partir du composé de formule (XV), la synthèse du composé de formule (XVI) mentionnée ci-dessus.

### a) Synthèse du composé de formule (XII)

Deux équivalents de formaldéhyde (0,9 mL ; 37% dans l'eau) sont ajoutés rapidement à une solution aqueuse de cyclame (1 g ; 1,07 mmoles dans 60 mL) à 0°C. Le mélange obtenu est placé sous agitation pendant 2 heures, moyennant quoi il se forme un précipité blanc, qui est ensuite filtré, lavé avec de l'eau et séché sous pression réduite. La poudre blanche obtenue est utilisé sans purification.

Le rendement est de 96%.

Les résultats de RMN ¹H et de RMN¹³C confirment l'obtention du composé de formule (XII).

### b) Synthèse du composé de formule (XIII)

600 mg du composé de formule (XII) sont dissous dans 70 mL d'éther diéthylique sec. Après 20 minutes de bullage sous argon, 10 mL d'une solution d'éther diéthylique contenant 75 µL de iodométhane (2,8 mmoles ; 1,05 équivalent) est ajoutée lentement. Après 24 heures sous agitation vigoureuse dans l'obscurité, il se forme un précipité blanc, qui est ensuite filtré et lavé avec 2*40 mL d'éther diéthylique. La poudre blanche obtenue est ensuite dissoute dans 100 mL de chloroforme, la solution étant filtrée pour éliminer les impuretés. La solution est évaporée sous vide, laissant subsister une huile incolore, qui cristallise lentement sous forme de plaquettes blanches. Le rendement est de 85%.

Les résultats de RMN¹³C confirment l'obtention du composé de formule (XIII).

### c) Synthèse du composé de formule (XIV)

A une solution d'acétonitrile comprenant le composé de formule (XIII) (1,47 g ; 4 mmoles dans 30 mL) est ajouté rapidement un excès d'iodobutyronitrile (1,48 g ; 7,6 mmoles). La solution obtenue est dégazée par bullage à l'argon et chauffée à 70°C pendant 30 minutes. La température est ensuite maintenue à 50°C durant 3 jours.

Il est obtenu un précipité blanc correspondant à un sel bis-ammonium de formule (XIV') suivante :

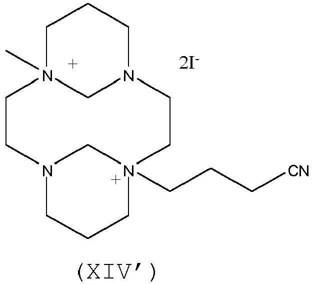

Ce précipité est filtré, lavé avec 2*50 mL d'acétonitrile, 50 mL de dichlorométhane et 100 mL d'éther diéthylique et la poudre blanche résultante est séchée sous pression réduite (1,43 g).

A partir du composé mentionné ci-dessus, le composé de formule (XIV) est obtenu de la manière suivante.

A une solution méthanolique du composé de formule (XIV') sous agitation magnétique, on ajoute un excès de borohydrure de sodium. Le mélange résultant est laissé à réagir pendant quelques heures. La solution est ensuite concentrée sous pression réduite et 50 mL d'eau distillée sont ajoutés. La solution aqueuse est ensuite extraite avec 3*50 mL de chloroforme. Les phases organiques sont réunies, séchées sur MgSO₄, évaporées et séchées sous pression réduite, moyennant quoi l'on obtient un résidu huileux incolore.

Les résultats de RMN ¹H et de RMN¹³C confirment l'obtention du composé de formule (XIV).

### d) Synthèse du composé de formule (XV)

A une solution éthanolique de composé de formule (XIV) sous agitation (0,3 g ; 0,97 mmoles dans 60 mL), on ajoute rapidement de l'hydroxyde de sodium (90 mg ; 2,21 mmoles) . A ce mélange sous agitation, on ajoute alternativement et par petites fractions du monohydrate d'hydrazine (700 µL ; 13 mmoles ; 15-20 équivalents) et du nickel Raney dans de l'éthanol (0,4 g dans 10 mL). Le mélange est laissé à réagir durant 24 heures puis filtré sur un filtre Millipore. Le filtrat est évaporé et le résidu est dissous dans un volume minimal de toluène. La solution est filtrée pour éliminer l'excès d'hydroxyde de sodium. Le filtrat est évaporé et séché sous pression réduite, moyennant quoi l'on obtient un résidu huileux incolore (Rendement : 0,279 g ; 92%).

Les résultats de RMN ¹H et de RMN¹³C confirment l'obtention du composé de formule (XV).

### e) Synthèse du composé de formule (XI)

A une solution sous agitation comprenant du dichlorométhane anhydre et de l'acide D,L-lipoïque (200 mg ; 0,96 mmoles dans 30 mL), préalablement dégazée à l'argon et refroidie à 0°C, on ajoute une solution de (2-(1H-7-azabenzotriazol-1-yl)-1,1,3,3-tétraméthyluronium hexafluorophosphate méthanaminium (0,366 g ; 0,96 mmoles) dissous dans 2 mL de diméthylformamide anhydre. On ajoute ensuite de la triéthylamine distillée (400 µL ; 2,88 mmoles) et le mélange est agité pendant 15 minutes à température ambiante. La solution est refroidie à 0°C et une solution comprenant du dichlorométhane anhydre et le composé de formule (XV) (278 mg ; 0,886 mmoles dans 35 mL) est ajoutée goutte à goutte. Le mélange obtenu est ensuite laissé sous agitation à température ambiante pendant 24 heures. Les solvants sont évaporés sous pression réduite et le résidu huileux obtenu est purifié par chromatographie sur colonne (alumine neutre, neutre, désactivée avec un mélange acétate d'éthyle/eau/méthanol (150/1/20)) avec de l'acétate d'éthyle pur comme solvant d'élution de départ puis des quantités croissantes de méthanol (5-20%) pendant l'élution. La fraction principale est évaporée jusqu'à siccité et le résidu huileux incolore obtenu est séché sous pression réduite (Rendement : 0,365 g ; 82%).

Les résultats de RMN¹³C confirment l'obtention du composé de formule (XI).

### EXEMPLE 2

Dans cet exemple est illustrée la fonctionnalisation d'une électrode en carbone vitreux.

Préalablement à cette fonctionnalisation, il est procédé à la préparation d'un complexe de cuivre de formule (IX) définie ci-dessus obtenu à partir du composé préparé selon l'exemple 1 de la façon exposée ci-dessous.

A une solution du composé (XI) (50 mg ; 9,97*10⁻⁵ moles) dissous dans 15 mL de méthanol sont ajoutés, sous agitation et à température ambiante, 32,6 mg de Cu(BF₄)₂.6H₂O (9,47*10⁻⁵ moles ; 0,95 équivalent) préalablement dissous dans 5 mL de méthanol. Après 30 minutes d'agitation, le solvant est évaporé sous pression réduite puis le résidu d'évaporation est lavé avec 2*10 mL de méthanol froid puis 50 mL d'éther diéthylique. Le résidu est alors dissous de nouveau dans 5 mL d'acétonitrile puis précipité par ajout de 30 mL d'éther diéthylique et filtré. Une poudre bleue hygroscopique est obtenue et est séchée sous vide. Le complexe est obtenu avec un rendement d'environ 80%.

Pour fonctionnaliser l'électrode en carbone vitreux, celle-ci est plongée dans une solution comprenant le complexe de cuivre de formule (IX), dont le mode de préparation est exposé ci-dessus, ce complexe étant présent à une concentration de 1 mM dans l'acétonitrile, et est ensuite soumise à une étape de cyclage entre -0,2 à +1 V/ Ag-Ag⁺ (10⁻² M dans l'acétonitrile) à raison d'une vitesse de balayage de 100 mV.s⁻¹, le nombre de cycles effectué étant de 5.

Le montage électrochimique utilisé est un montage à trois électrodes avec un électrolyte organique comprenant :
- une électrode de référence consistant en un fil d'argent massif mis en contact avec une solution de AgNO₃ (10⁻² M) dans l'acétonitrile ;
- une électrode de travail consistant en un disque de carbone vitreux de 3 mm de diamètre ;
- une contre-électrode consistant en un fil de platine ; et
- un électrolyte consistant en de l'acétonitrile contenant du perchlorate de tétrabutylammonium (0,1 M) et du complexe de cuivre de formule (IX) (1 mM).

Suite à cette fonctionnalisation et après rinçage avec l'acétonitrile, l'électrode ainsi modifiée est soumise à une expérience de voltampérométrie cyclique consistant à soumettre le substrat ainsi revêtu à des cycles successifs (au nombre de 150) entre -1 V et + 0,1 V par rapport à Ag/Ag⁺ (10⁻² M) à raison de 50 V.s⁻¹.

Le montage utilisé comprend :
- une électrode de référence consistant en un fil d'argent massif mis en contact avec une solution de AgNO₃ (10⁻² M) dans l'acétonitrile ;
- une électrode de travail consistant en le substrat revêtu ;
- une contre-électrode consistant en un fil de platine ; et
- un électrolyte consistant en de l'acétonitrile contenant du perchlorate de tétrabutylammonium (0,1 M).

Le voltammogramme obtenu est représenté sur la figure 2 jointe en annexe.

Pour le cycle 1 indiqué sur la figure, on peut constater un système redox réversible (centré autour de -0,7 V) attribué au système redox du Cu^{II} pour le composé greffé dans une configuration diastéréoisomérique du type I, correspondant au diastéréoisomère RSRS.

Au fur et à mesure des cycles, ce système redox disparaît au profit d'un nouveau système redox réversible (centré autour de -0,25 V) attribuable au système redox du Cu^{II} pour le composé greffé dans une configuration diastéréoisomérique du type V (correspondant au diastéréoisomère RRRR), comme cela est illustré pour le cycle 150 représenté sur la même figure 2.

Cela indique, sans ambiguïté, que les réactions de transfert d'électrons du complexe immobilisé sur une surface sont couplées à des réactions chimiques et que le passage d'un état à l'autre est réalisé. Les deux formes stables du « switch » présentent des potentiels d'oxydoréduction séparés de 500 mV, ce qui atteste d'une stabilisation des différents états redox.

Dans l'optique d'un stockage d'informations, le fait de pouvoir identifier les deux isomères (type I et type V) à une vitesse de balayage élevée signifie que l'on pourra lire les deux informations correspondantes sans trop de perte d'informations. En d'autres termes, il est possible de lire l'information (« 0 » ou « 1 ») sans transformer le « 0 » en « 1 » et inversement.

## Revendications

1. Procédé de fonctionnalisation d'un substrat conducteur de l'électricité qui n'est pas un substrat en or par une couche de composés chimiques, comprenant les étapes suivantes :
- une étape de mise en contact du substrat conducteur de l'électricité avec des composés chimiques comprenant au moins un groupe terminal disulfure;
- une étape d'électrooxydation du groupe terminal disulfure desdits composés chimiques moyennant quoi lesdits composés chimiques forment une couche à la surface du substrat conducteur de l'électricité, dans lequel lesdits composés chimiques, outre la présence d'au moins un groupe terminal disulfure, comprennent au moins un groupe de stockage de charges, ledit groupe de stockage de charges et ledit groupe terminal étant séparés éventuellement par au moins un groupe espaceur organique, lesdits composés chimiques répondant à la formule générale (I) suivante :
X-[-L-Z]ₙ (I)
dans laquelle :
- X représente un groupe de stockage de charges, qui est un groupe tétraazacycloalcane complexé avec au moins un élément métallique, le groupe tétrazacycloalcane répondant à l'une des formules (II), (III) et (IV) suivantes : 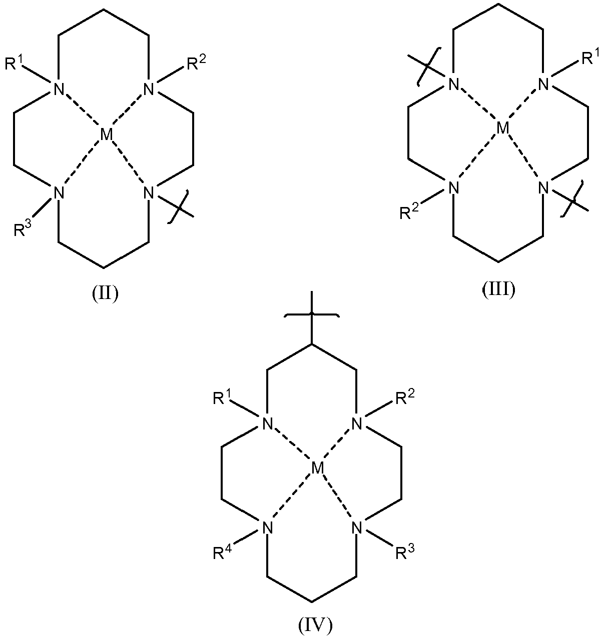 dans lesquelles :
- R¹, R², R³ et R⁴ représentent, indépendamment l'un de l'autre, un groupe alkyle, aryle, alkylaryle, un atome d'halogène, un groupe (alkyl)métallocène, lesdits groupes pouvant être perfluorés ;
- M est un élément métallique présentant au moins deux degrés d'oxydation,
les accolades indiquant l'endroit par lequel ces groupes sont liés au groupe disulfure via éventuellement un groupe espaceur organique ;
- L représente une liaison simple ou un groupe espaceur organique ;
- Z représente un groupe disulfure cyclique ; et
- n est un entier allant de 1 à 6.

2. Procédé de fonctionnalisation d'un substrat conducteur de l'électricité selon la revendication 1, dans lequel l'élément métallique est choisi parmi les éléments métalliques de transition.

3. Procédé de fonctionnalisation d'un substrat conducteur de l'électricité selon la revendication 2, dans lequel l'élément métallique de transition est choisi parmi Cr, Mn, Fe, Co, Ni, Cu.

4. Procédé de fonctionnalisation d'un substrat conducteur de l'électricité selon l'une quelconque des revendications 1 à 3, dans lequel le groupe espaceur organique est un groupe hydrocarboné, se présentant sous forme d'une chaîne linéaire ou ramifiée, dans laquelle peuvent venir s'intercaler un ou plusieurs groupes de liaison.

5. Procédé de fonctionnalisation selon l'une quelconque des revendications 1 à 4, dans lequel le groupe espaceur organique répond à la formule (VII) suivante :
-(CH₂)₄-NH-CO-(CH₂)₄- (VII)

6. Procédé de fonctionnalisation selon l'une quelconque des revendications précédentes, dans lequel le groupe disulfure est un groupe de formule (VIII) suivante :

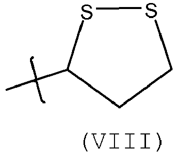

7. Procédé de fonctionnalisation selon l'une quelconque des revendications précédentes, dans lequel les composés organiques répondent à l'une des formules (IX) et (X) suivantes : 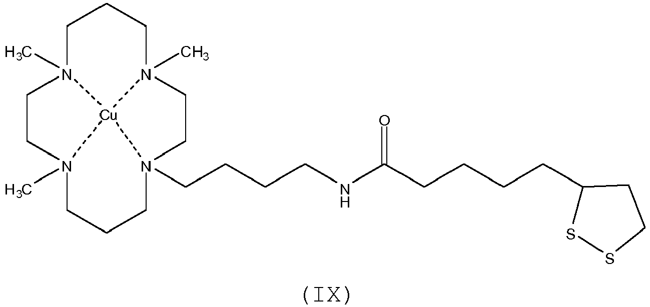 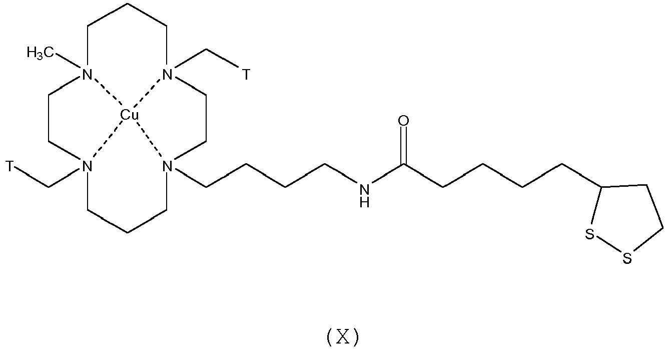 avec T représentant un groupe ferrocène.

8. Substrat fonctionnalisé susceptible d'être obtenu par un procédé tel que défini selon l'une quelconque des revendications 1 à 7.

9. Cellule de mémoire comprenant au moins un substrat fonctionnalisé tel que défini à la revendication 8.

10. Cellule de mémoire selon la revendication 9, qui est une cellule capacitive de mémoire.

11. Composé organique comprenant au moins un groupe de stockage de charges et au moins un groupe terminal disulfure cyclique, ledit groupe de stockage de charges et ledit groupe disulfure étant éventuellement séparés par un groupe espaceur organique, ledit composé chimique répondant à la formule générale (I) suivante :
X-[-L-Z]ₙ (I)
dans laquelle :
- X représente un groupe de stockage de charges, qui est un groupe tétraazacycloalcane complexé avec au moins un élément métallique, le groupe tétrazacycloalcane répondant à l'une des formules (II), (III) et (IV) suivantes : 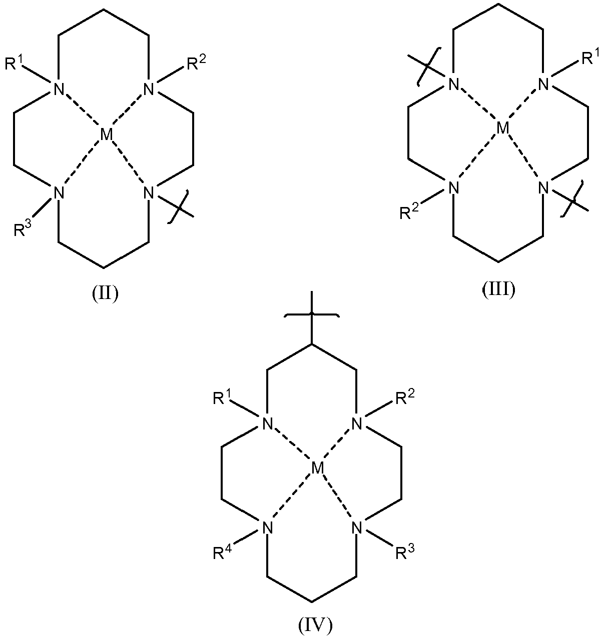 dans lesquelles :
- R¹, R², R³ et R⁴ représentent, indépendamment l'un de l'autre, un groupe alkyle, aryle, alkylaryle, un atome d'halogène, un groupe (alkyl)métallocène, lesdits groupes pouvant être perfluorés ;
- M est un élément métallique présentant au moins deux degrés d'oxydation,
les accolades indiquant l'endroit par lequel ces groupes sont liés au groupe disulfure via éventuellement un groupe espaceur organique ;
- L représente une liaison simple ou un groupe espaceur organique ;
- Z représente un groupe disulfure cyclique ; et
- n est un entier allant de 1 à 6.

## Patentansprüche

1. Verfahren zur Funktionalisierung eines elektrisch leitenden Substrats, das kein Substrat aus Gold ist, durch eine Schicht von chemischen Verbindungen, umfassend die folgenden Schritte:
- einen Schritt des Inkontaktbringens des elektrisch leitenden Substrats mit chemischen Verbindungen, die wenigstens eine Disulfidendgruppe umfassen;
- einen Schritt der Elektrooxidation der Disulfidendgruppe der chemischen Verbindungen, wodurch die chemischen Verbindungen eine Schicht auf der Oberfläche des elektrisch leitenden Substrats bilden, wobei die chemischen Verbindungen, abgesehen vom Vorhandensein wenigstens einer Disulfidendgruppe wenigstens eine Ladungsspeicherungsgruppe umfassen, wobei die Ladungsspeicherungsgruppe und die Endgruppe gegebenenfalls durch wenigstens eine organische Spacergruppe separiert sind, wobei die chemischen Verbindungen der nachfolgenden allgemeinen Formel (I) genügen:
X-[-L-Z]ₙ (I)
wobei:
- X eine Ladungsspeicherungsgruppe repräsentiert, die eine Tetraazazykloalkangruppe ist, komplexiert mit wenigstens einem metallischen Element, wobei die Tetraazazykloalkangruppe einer der nachfolgenden Formeln (II), (III) und (IV) genügt: 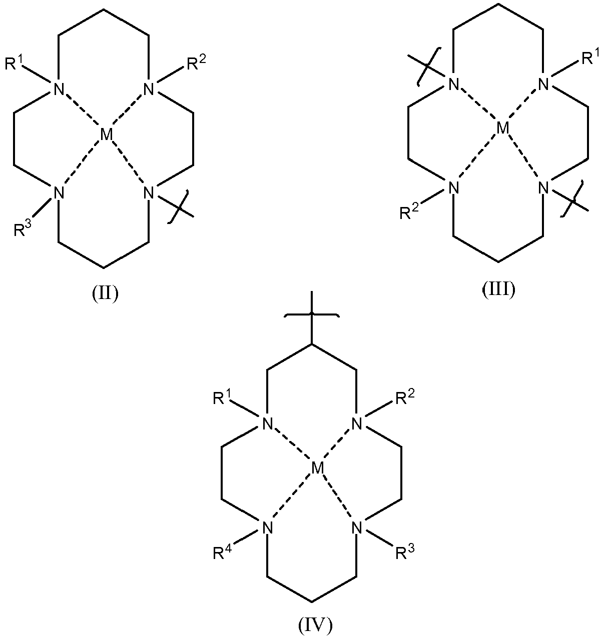 wobei:
- R¹, R², R³ und R⁴ unabhängig voneinander eine Alkyl-, Aryl-, Alkylaryl-Gruppe, ein Halogenatom, eine (Alkyl)Metallozengruppe repräsentieren, wobei die Gruppen perfluoriert sein können;
- M ein metallisches Element ist, das wenigstens zwei Oxidationsstufen aufweist, wobei die Klammern den Ort angeben, an dem diese Gruppen mit der Disulfidgruppe verbunden sind, gegebenenfalls über eine organische Spacergruppe;
- L eine Einfachbindung oder eine organische Spacergruppe repräsentiert;
- Z eine zyklische Disulfidgruppe repräsentiert; und
- n eine ganze Zahl ist, die von 1 bis 6 geht.

2. Verfahren zur Funktionalisierung eines elektrisch leitenden Substrats nach Anspruch 1, wobei das metallische Element ausgewählt ist aus den metallischen Übergangselementen.

3. Verfahren zur Funktionalisierung eines elektrisch leitenden Substrats nach Anspruch 2, wobei das metallische Übergangselement ausgewählt ist aus Cr, Mn, Fe, Co, Ni, Cu.

4. Verfahren zur Funktionalisierung eines elektrisch leitenden Substrats nach einem der Ansprüche 1 bis 3, wobei die organische Spacergruppe eine Kohlenwasserstoffgruppe ist, die die Form einer linearen oder verzweigten Kette aufweist, in die ein oder mehrere Verbindungsgruppen interkaliert sein können.

5. Verfahren zur Funktionalisierung nach einem der Ansprüche 1 bis 4, wobei die organische Spacergruppe der nachfolgenden Formel (VII) genügt:
-(CH₂)₄-NH-CO-(CH₂)₄- (VII) .

6. Verfahren zur Funktionalisierung nach einem der vorhergehenden Ansprüche, wobei die Disulfidgruppe eine Gruppe der nachfolgenden Formel (VIII) ist:

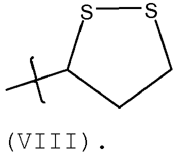

7. Verfahren zur Funktionalisierung nach einem der vorhergehenden Ansprüche, wobei die organischen Verbindungen einer der nachfolgenden Formeln (IX) und (X) genügen: 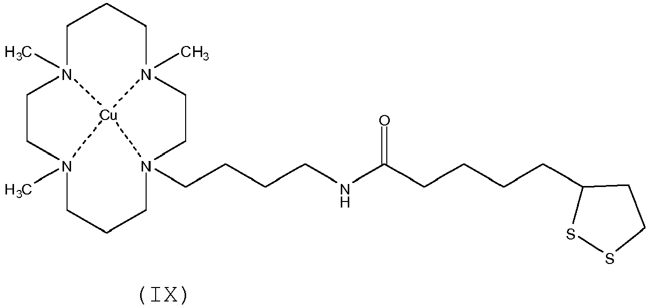 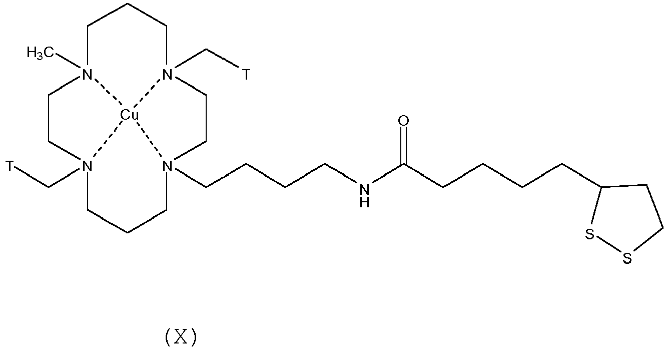 wobei T eine Ferrozengruppe repräsentiert.

8. Funktionalisiertes Substrat, das erhältlich ist durch ein Verfahren wie in einem der Ansprüche 1 bis 7 definiert.

9. Speicherzelle, umfassend wenigstens ein funktionalisiertes Substrat wie in Anspruch 8 definiert.

10. Speicherzelle nach Anspruch 9, die eine kapazitive Speicherzelle ist.

11. Organische Verbindung, umfassend wenigstens eine Ladungsspeicherungsgruppe und wenigstens eine zyklische Disulfidendgruppe, wobei die Ladungssspeicherungsgruppe und die Disulfidgruppe gegebenenfalls durch eine organische Spacergruppe separiert sind, wobei die chemische Verbindung der nachfolgenden allgemeinen Formel (I) genügt:
X-[-L-Z]ₙ (I)
wobei:
- X eine Ladungsspeicherungsgruppe repräsentiert, die eine Tetraazazykloalkangruppe ist, komplexiert mit wenigstens einem metallischen Element, wobei die Tetraazazykloalkangruppe einer der nachfolgenden Formeln (II), (III) und (IV) genügt: 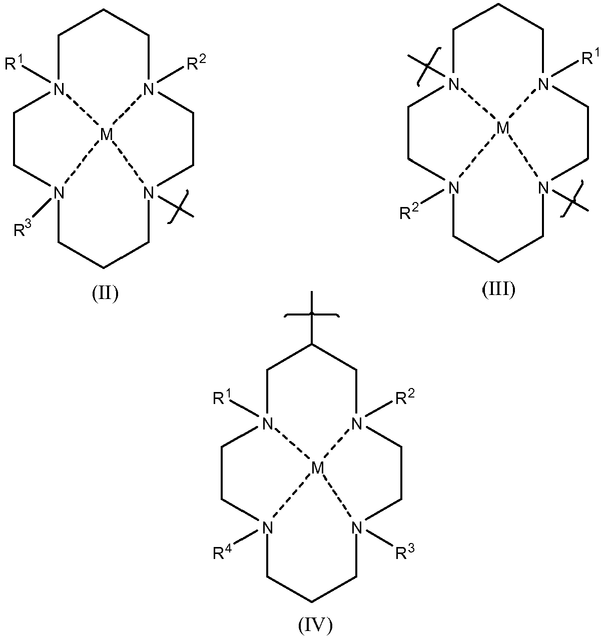 wobei:
- R¹, R², R³ und R⁴ unabhängig voneinander eine Alkyl-, Aryl-, Alkylaryl-Gruppe, ein Halogenatom, eine (Alkyl)Metallozengruppe repräsentieren, wobei die Gruppen perfluoriert sein können;
- M ein metallisches Element ist, das wenigstens zwei Oxidationsstufen aufweist, wobei die Klammern den Ort angeben, an dem diese Gruppen mit der Disulfidgruppe verbunden sind, gegebenenfalls über eine organische Spacergruppe;
- L eine Einfachbindung oder eine organische Spacergruppe repräsentiert;
- Z eine zyklische Disulfidgruppe repräsentiert; und
- n eine ganze Zahl ist, die von 1 bis 6 geht.

## Claims

1. Method for functionalizing an electrically conductive substrate, which is not a substrate made of gold, via a layer of chemical compounds, comprising the following steps:
- a step in which the electrically conductive substrate is placed in contact with chemical compounds comprising at least a disulfide terminal group;
- a step in which the disulfide terminal group of said chemical compounds is electro-oxidized, causing said chemical compounds to form a layer at the surface of the electrically conductive substrate, wherein said chemical compounds, apart from the presence of at least a disulfide terminal group, comprise at least a charge storage group, said charge storage group and said disulfide terminal group being separated potentially by at least an organic spacer group, said chemical compounds meeting the following generic formula (I):
X-[-L-Z]ₙ (I)
in which:
- X represents a charge storage group, which is a tetraazacycloalkane group complexed with at least a metal element, the tetraazacycloalkane group meeting one of the following formulas (II), (III) and (IV): 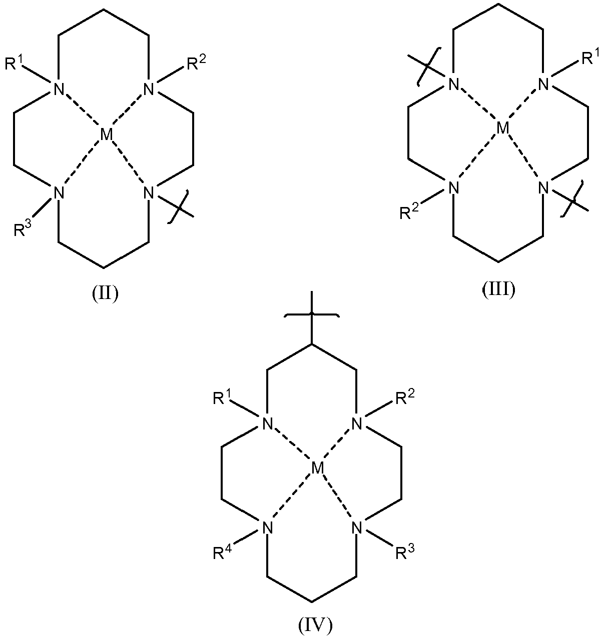 in which:
- R¹, R², R³ and R⁴ represent, independently of each other, an alkyl, aryl, alkylaryl group, a halogen atom, an (alkyl)metallocene group, said groups being able to be perfluorinated;
- M is a metal element having at least two degrees of oxidation,
the braces indicating the spot via which said groups are bound to the disulfide group via potentially an organic spacer group;
- L represents a single bond or an organic spacer group;
- Z represents a disulfide group; and
- n is a whole number ranging from 1 to 6.

2. Method for functionalizing an electrically conductive substrate according to claim 1, in which the metal element is selected from transition metal elements.

3. Method for functionalizing an electrically conductive substrate according to claim 2, in which the transition metal element is selected from Cr, Mn, Fe, Co, Ni, Cu.

4. Method for functionalizing an electrically conductive substrate according to any of claims 1 to 3, in which the organic spacer group is a hydrocarbon group, being in the form of a linear or branched chain, in which one or more bonding groups can be inserted.

5. Method for functionalizing according to any of claims 1 to 4, in which the organic spacer group meets the following formula (VII):
-(CH₂)₄-NH-CO-(CH₂)₄- (VII)

6. Method for functionalizing according to any of the preceding claims, in which the disulfide group is a group of following formula (VIII):

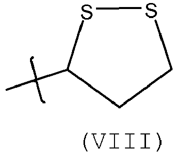

7. Method for functionalizing according to any of the preceding claims, in which the organic compounds meet one of the following formulas (IX) and (X) : 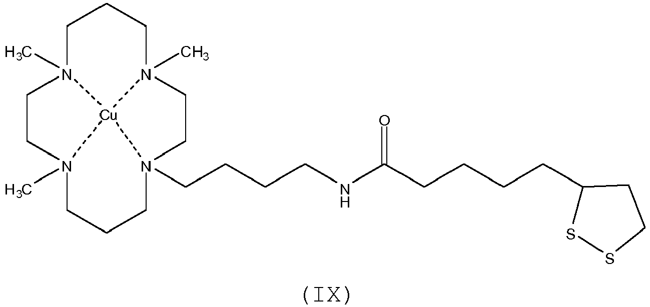 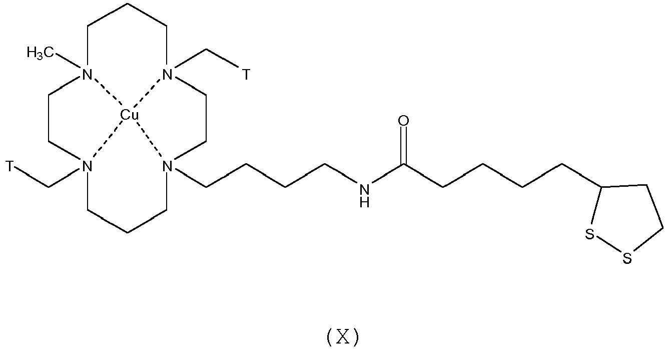 with T represents a ferrocene group.

8. Functionalized substrate capable of being obtained by a method as defined according to any of claims 1 to 7.

9. Memory cell comprising at least a functionalized substrate as defined in claim 8.

10. Memory cell according to claim 9, which is a capacitive memory cell.

11. Organic compound comprising at least a charge storage group and at least a cyclic disulfide terminal group, said charge storage group and said disulfide group being potentially separated by an organic spacer group, said organic compound meeting the following generic formula (I):
X-[-L-Z]ₙ (I)
in which:
- X represents a charge storage group, which is a tetraazacycloalkane group meeting one of the following formulas (II), (III) and (IV): 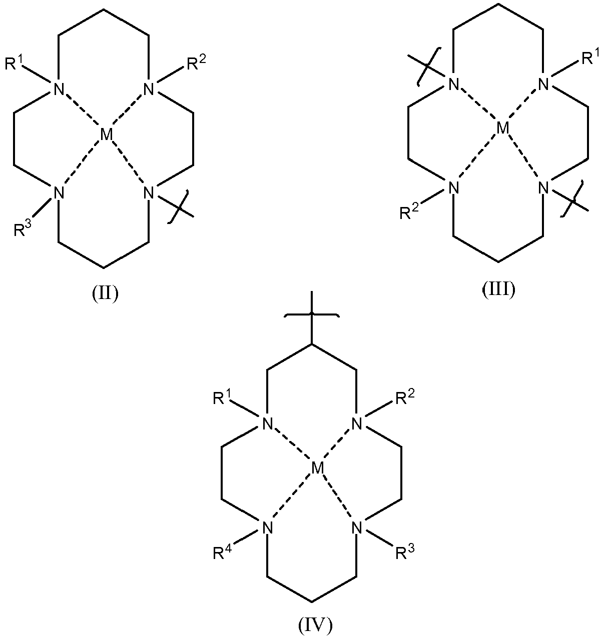 in which:
- R¹, R², R³ and R⁴ represent, independently of each other, an alkyl, aryl, alkylaryl group, a halogen atom, an (alkyl)metallocene group, said groups being able to be perfluorinated;
- M is a metal element having at least two degrees of oxidation,
the braces indicating the spot via which said groups are bound to the disulfide group via potentially an organic spacer group;
- L represents a single bond or an organic spacer group;
- Z represents a disulfide group; and
- n is a whole number ranging from 1 to 6.
